# FASCICULE DE BREVET EUROPEEN

(11) **EP 2 084 355 B1**
(45) Date de publication et mention de la délivrance du brevet: **02.05.2012**
(21) Numéro de dépôt: 07822124.9
(22) Date de dépôt: 31.10.2007
(51) Int. Cl.: E05B 73/00, A61B 6/00, G06F 1/16

(54) **DISPOSITIF ANTIVOL POUR UN EQUIPEMENT PORTABLE**
SCHLOSS FÜR EINE TRAGBARE VORRICHTUNG
LOCK FOR A PORTABLE DEVICE

(30) Priorité: 02.11.2006 FR 0654695
(43) Date de publication de la demande: 05.08.2009
(73) Titulaire: Trixell S.A.S., 38430 Moirans (FR)
(72) Inventeur: WIRTH, Thibaut, 38500 Coublevie (FR)
(74) Mandataire: Lucas, Laurent Jacques
(86) Numéro de dépôt international: PCT/EP2007/061777
(87) Numéro de publication internationale: WO 2008/053022

(56) Documents cités:
- US-A- 5 841 631
- US-A1- 2003 161 099
- US-B1- 6 307 738

## Description

La présente invention concerne une station d'accueil, selon le préambule de la revendication 1.

Dans le domaine de l'imagerie médicale l'évolution des équipements d'imagerie vers des équipements légers et portables génère un besoin nouveau en matière de protection contre le vol de ces appareils. Par exemple un capteur de rayons X portable sans fil est un objet très coûteux que l'on peut facilement dérober. Un système anti-vol pour un tel capteur doit répondre à deux critères principaux qui sont :
■ décourager les voleurs potentiels par la difficulté technique du vol,
■ permettre au capteur de rester utilisable par plusieurs personnes en limitant les contraintes d'utilisation.

Jusqu'à présent, les seuls systèmes antivols utilisés sont du type de ceux utilisés pour les ordinateurs portables : un câble généralement métallique fixé à l'ordinateur portable et relié à un élément plus imposant, comme par exemple une table, avec pour seul moyen de déblocage une clef mécanique.
Les principaux inconvénients de ces antivols sont les suivants :
■ une action mécanique et manuelle est nécessaire pour verrouiller l'anti-vol avec le risque que le personnel dépose le capteur sans penser à effectuer le verrouillage,
■ le déblocage de l'antivol nécessite d'être en possession de la clef, ce qui implique d'avoir la clef en permanence sur soi avec un risque de perte non négligeable,
■ l'utilisation par plusieurs personnes implique une multiplication des clefs et présente donc un risque accru de perte de ces clefs et éventuellement un risque de confusion entre différentes clefs ou un risque d'utilisation malhonnête de la clef si cette dernière tombe entre de mauvaises mains.

La demande de brevet DE10202330 décrit un système de verrouillage pour les portes d'un véhicule automobile utilisant une commande de déblocage des serrures électrique à partir d'un signal électrique.

Un but de l'invention est notamment de pallier les inconvénients précités. A cet effet, l'invention a pour objet une station d'accueil pour un équipement portable capteur de rayons X selon la revendication 1.

La mise à l'état inhibé du signal électrique peut être réalisée à partir d'un message d'inhibition envoyé par l'unité logicielle à l'équipement d'accueil. Le message d'inhibition peut produire un signal, amplifié par une interface adaptée. Le signal amplifié ouvre un circuit d'alimentation du système électromécanique.

Une désinhibition du signal électrique peut être réalisée à partir d'un message de désinhibition envoyé par l'unité logicielle à l'équipement d'accueil. Le message de désinhibition produit un signal amplifié par une interface adaptée. Le signal amplifié ferme le circuit d'alimentation du système électromécanique.

La saisie d'un mot de passe par l'intermédiaire de l'unité logicielle peut entraîner l'envoi du message de désinhibition par l'unité logicielle. La saisie d'un mot de passe sécurise l'utilisation de l'équipement portable.

L'envoi du message d'inhibition peut être effectué automatiquement par l'unité logicielle en respectant un critère temporel paramétrable.

Le système électromécanique peut comporter au moins un électroaimant agissant sur un aimant relié aux moyens de fixation et permettant l'ouverture des moyens de fixation.

Le signal électrique est produit par exemple par un bouton disposé sur l'équipement d'accueil.

Le système électromécanique peut comporter un moyen de déblocage mécanique.

L'unité logicielle peut être un ordinateur, comportant un logiciel de gestion de système d'imagerie médicale, relié à la station d'accueil par une liaison.

L'invention a notamment pour principaux avantages d'être simple à utiliser et à mettre en oeuvre ainsi que peu coûteuse à réaliser.

D'autres caractéristiques et avantages de l'invention apparaîtront à l'aide de la description qui suit, donnée à titre illustratif et non limitatif, et faite en regard des dessins annexés qui représentent :
- la figure 1 : un schéma général d'un système d'imagerie médicale,
- la figure 2a : une représentation schématique du dispositif de fixation d'un capteur sur une station d'accueil en position débloquée,
- la figure 2b : une représentation schématique du dispositif de fixation du capteur sur une station d'accueil en position bloquée,
- la figure 3: une représentation schématique du dispositif de blocage / déblocage du capteur sur la station d'accueil,
- la figure 4 : une vue synoptique du fonctionnement du dispositif de blocage / déblocage du capteur suivant différentes configurations.

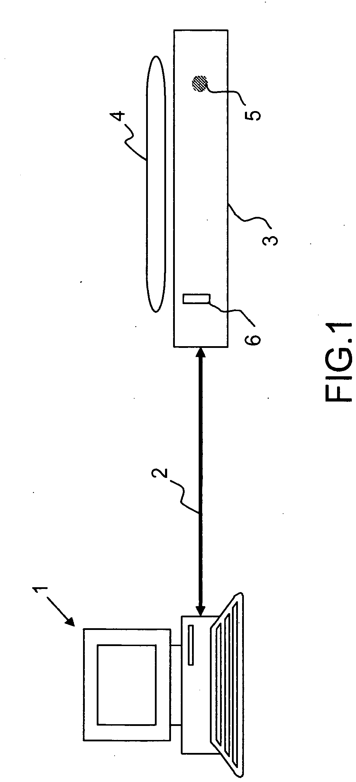

La figure 1 représente un système d'imagerie médicale. Ce système comporte une unité logicielle 1 de gestion du système d'imagerie médicale. Cette unité logicielle 1 peut être un ordinateur 1 comportant un logiciel de gestion du système d'imagerie médicale. L'ordinateur 1 communique par une liaison 2 avec une station d'accueil 3 pour un capteur 4. La liaison 2 entre la station d'accueil 3 et l'ordinateur 1 peut être une liaison filaire ou optique ou tout autre type de liaison permettant un échange de données entre deux équipements électroniques. La station d'accueil 3 peut être par exemple une station de recharge des batteries du capteur 4 servant également au capteur 4 de relais de communication vers l'ordinateur 1 de gestion du système d'imagerie médicale. Le capteur 4 au repos est donc posé sur la station d'accueil 3. La station d'accueil 3 possède un dispositif de fixation du capteur 4 lorsque celui-ci est posé à son contact. Ce dispositif de fixation est associé à un dispositif selon l'invention de blocage et de déblocage du capteur 4 lorsqu'il est posé sur la station d'accueil 3. Le déblocage du capteur 4 peut se faire soit à l'aide d'un bouton latéral 5, qui peut être un bouton poussoir, soit à l'aide d'une clef à introduire dans une serrure 6 positionnée sur la station d'accueil 3.

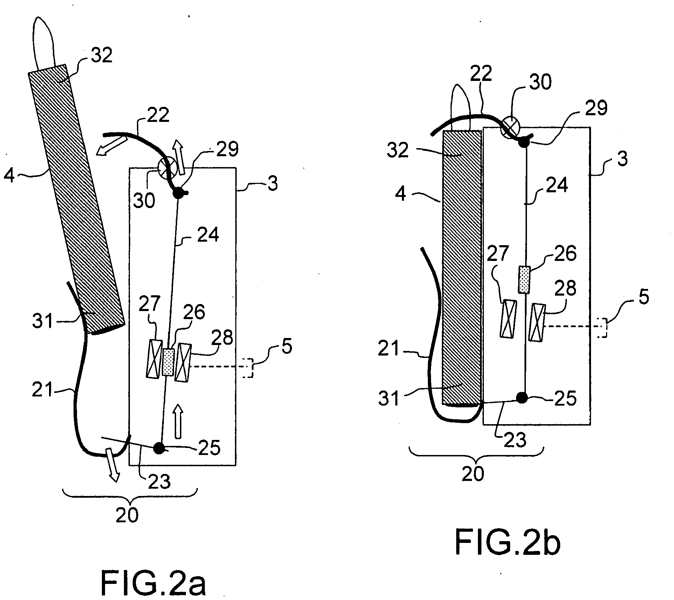

Les figures 2a et 2b représentent un exemple de dispositif de fixation du capteur 4 sur la station d'accueil 3. La figure 2a représente le capteur 4 dans une position non bloquée par rapport à la station d'accueil 3. La figure 2b représente quant à elle le capteur 4 bloqué sur la station d'accueil 3. Le capteur 4, sur les figures 2a et 2b, se présente sous la forme d'une petite valise, comportant une première extrémité 31 et une deuxième extrémité 32, que l'on pose sur la station d'accueil 3. La station d'accueil 3, représentée sous la forme d'un bloc rectangulaire, est pourvue d'un dispositif de fixation 20 permettant un maintien du capteur 4 lorsque ce dernier est posé sur la station d'accueil 3. Le dispositif de fixation 20 du capteur 4 sur la station d'accueil 3 est, dans le cadre du dispositif selon l'invention, bloqué lorsque le capteur 4 est posé sur la station d'accueil 3 afin que le capteur 4 ne puisse être enlevé de la station d'accueil 3 sans effectuer une procédure spécifique de déblocage. Un bouton 5, positionné par exemple sur la partie latérale de la station d'accueil 3, peut servir à libérer le capteur 4 par une simple pression. Cette pression permet d'actionner le dispositif de fixation 20 afin qu'il libère le capteur 4. Le bouton 5 peut être placé de manière non évidente afin que seul le personnel ayant connaissance de son existence puisse l'actionner. Ceci permet de réduire le nombre de personnes en mesure d'ôter le capteur 4 de la station d'accueil 3.

Le dispositif de fixation 20 du capteur 4 sur la station d'accueil 3 peut comporter deux lames rigides 21, 22 permettant de maintenir le capteur 4 en contact avec la station d'accueil 3. Le capteur 4 est glissé entre la première lame 21 et la station d'accueil 3 afin de se retrouver plaqué sur la station d'accueil 3. La première lame 21 est solidaire de la station d'accueil 3. Lorsqu'une première extrémité 31 du capteur 4 est positionnée entre la première lame 21 et la station d'accueil 3, le capteur 4 exerce une pression sur une première tige 23 reliée à une deuxième tige 24 par un premier axe mobile de rotation 25. Le mouvement de rotation de la première tige 23 entraîne une translation de la deuxième tige 24. Un aimant 25 est monté sur la deuxième tige 24. Lorsque le dispositif de fixation 20 du capteur 4 est dans une position ouverte comme représenté sur la figure 2a, l'aimant 26 se trouve positionné entre deux électroaimants 27, 28. Lorsque le dispositif de fixation 20 du capteur 4 se ferme, l'aimant 26 se trouve entraîné en translation par la deuxième tige 24 : il n'est plus positionné entre les deux électroaimants 27, 28. La deuxième tige 24, lors de la fermeture du dispositif de fixation 20 du capteur 4, entraîne dans son mouvement de translation un deuxième axe mobile de rotation 29 solidaire de la deuxième tige 24 et de la deuxième lame 22. La translation de la deuxième tige 24 va entraîner, par l'intermédiaire du deuxième axe de rotation 29, une rotation de la deuxième lame 22 autour d'un troisième axe de rotation 30 solidaire de la station d'accueil 3. La deuxième lame 22 va alors venir coiffer la deuxième extrémité 32 du capteur 4, effectuant ainsi un blocage du capteur 4 sur la station d'accueil 3.

Lorsque le dispositif de fixation 20 du capteur 4 est fermé, une pression sur le bouton 5 permet d'alimenter les électroaimants 27, 28 qui attirent l'aimant 26 créant ainsi une translation de la tige 24 dans le sens opposé à la translation utilisée pour la fermeture du dispositif de fixation 20 du capteur 4. Cette translation entraîne une rotation de la deuxième lame 22 autour du troisième axe de rotation 30, libérant ainsi le capteur 4.

Le dispositif de fixation 20 permet avantageusement un blocage du capteur 4 de manière automatique lors du dépôt de celui-ci sur la station d'accueil 3. La prise en main du capteur 4 nécessite une opération manuelle simple qui peut consister à presser le bouton 5 puis à retirer le capteur 4 du dispositif de fixation 20 ouvert grâce à la pression sur le bouton 5.

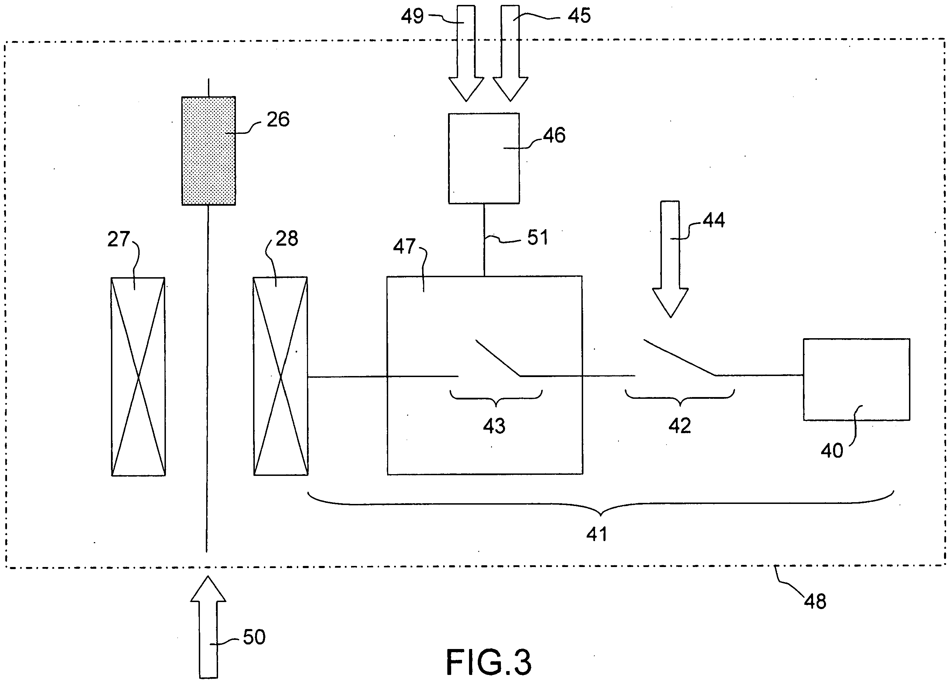

La figure 3 présente une mise en oeuvre d'un dispositif électromécanique 48 selon l'invention de blocage et de déblocage du capteur 4 lorsqu'il est positionné sur la station d'accueil 3, bloqué par le dispositif de fixation 20 du capteur 4. Le dispositif 48 de blocage et de déblocage selon l'invention peut par exemple comporter une alimentation 40 permettant d'alimenter les électroaimants 27, 28 lorsque le circuit d'alimentation 41 est fermé. Le circuit d'alimentation 41 possède par exemple deux interrupteurs 42, 43. Le premier interrupteur 42 se ferme sur une pression du bouton 5 qui génère un signal électrique 44 à cet effet. La fermeture du premier interrupteur 42 provoque une alimentation électrique des deux électroaimants 27, 28, débloquant ainsi le capteur 4 comme représenté sur la figure 2a. Le déblocage du capteur réalisé au moyen du bouton 5 est donc un déblocage électrique.

Le deuxième interrupteur 43 est ouvert uniquement lorsqu'une inhibition du déblocage électromagnétique à été demandée. L'inhibition du déblocage du capteur 4, à l'aide du bouton 5, peut être réalisée au moyen d'un message d'inhibition 45 envoyé de l'ordinateur 1 vers la station 3. Une fois ce message d'inhibition 45 reçu par la station d'accueil 3, une interface adaptée 46 transforme ce message en un signal amplifié 51 transmis à un boîtier de commande 47 qui ouvre l'interrupteur 43. Dans ce cas, une pression sur le bouton 5 n'a aucun effet sur le déblocage du capteur 4 et ceci tant qu'un message de désinhibition 49 n'a pas été envoyé par l'ordinateur 1.

Lorsque la station d'accueil 3 reçoit un message de désinhibition 49, l'interface 46 transforme ce message de désinhibition 49 en un signal amplifié 51 transmis au boîtier de commande 47 afin qu'il ferme l'interrupteur 43. Une fois l'interrupteur 43 fermé, une simple pression sur le bouton 5 permet de débloquer le capteur 4.

Le message d'inhibition 45 peut être envoyé par un utilisateur via une interface homme machine du logiciel de gestion du système d'imagerie médicale. Le message d'inhibition 45 peut également être envoyé automatiquement par le logiciel de gestion du système d'imagerie médicale sur un critère paramétrable dans le logiciel de gestion du système d'imagerie médicale. Ce critère peut être par exemple un critère temporel comme une heure et une date de début de l'inhibition ou bien un laps de temps déterminé depuis la dernière utilisation du capteur 4. Le fonctionnement de l'inhibition en mode automatique ou manuel peut être paramétrable dans le logiciel de gestion du système d'imagerie médicale. Le message de désinhibition 49 peut être envoyé sur une action de l'utilisateur à partir du logiciel de gestion du système d'imagerie médicale ou bien en entrant un mot de passe par exemple. Ceci permet de sécuriser l'utilisation de l'équipement portable : en effet seules les personnes ayant une connaissance du logiciel de gestion du système d'imagerie médicale ou bien encore du mot de passe peuvent désinhiber le déblocage du capteur 4.

Lorsque le message de désinhibition 49 du déblocage ne peut être envoyé, le déblocage électrique par le bouton 5 ayant été mis à l'état inhibé, un moyen mécanique 50 de déblocage permet par exemple au moyen d'une clef, à faire jouer dans la serrure 6 de la station d'accueil 3, de libérer le capteur 4 de la station 3. La clef peut agir par exemple en entraînant un système mécanique non représenté qui permet de tirer la tige 24 afin de positionner l'aimant 26 entre les deux électroaimants 27, 28, ce qui entraîne le déblocage du capteur 4 comme représenté sur la figure 2a. Ceci permet donc avantageusement de pouvoir débloquer le capteur 4 lorsque la liaison 2 entre l'ordinateur et la station d'accueil 3 ne fonctionne pas ou que le mot de passe de l'ordinateur a été perdu par exemple.

Le dispositif selon l'invention permet donc de disposer de plusieurs moyens de blocage et de déblocage du capteur 4 suivant différents cas d'utilisation.

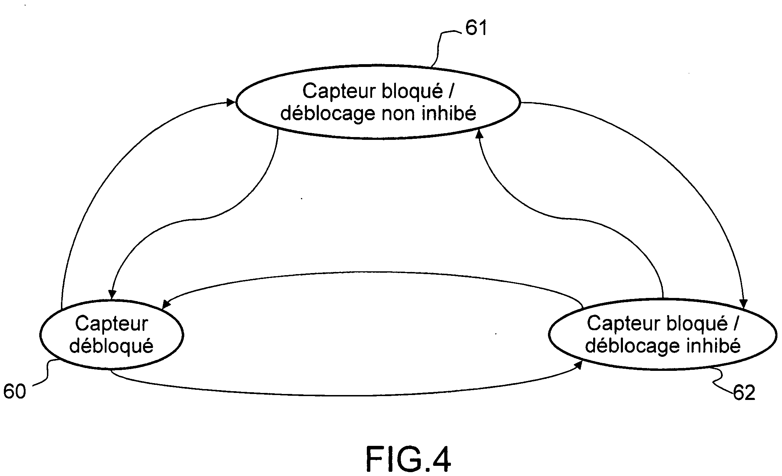

La figure 4 représente un synoptique des procédures à effectuer afin de libérer le capteur 4 selon l'état de blocage de ce dernier. Le principe général du dispositif selon l'invention est de pouvoir utiliser le dispositif d'antivol d'au moins trois manières différentes :
■ en utilisation standard, une pression sur le bouton 5 de la station 3 suffit à débloquer le capteur 4 afin de pouvoir l'ôter de la station d'accueil 3,
■ dans un mode d'utilisation plus sécurisé une inhibition de l'action de déblocage est réalisée au moyen du bouton 5,
■ dans un mode dégradé d'utilisation, en cas de disfonctionnement par exemple, il est possible de libérer le capteur à l'aide d'une clef.
Le capteur 4 peut être dans plusieurs états relatifs à son blocage sur la station 3 : un premier état peut être celui du capteur débloqué 60 lorsqu'il est en dehors de la station d'accueil par exemple ou que le dispositif de fixation 20 ne bloque pas le capteur 4 sur la station d'accueil 3. Un second état 61 peut correspondre à la situation dans laquelle le capteur 4 est bloqué sur la station d'accueil 3 mais que le déblocage n'est pas inhibé, c'est par exemple typiquement le cas de l'utilisation standard du dispositif antivol : il suffit alors d'une pression sur le bouton 5 pour libérer le capteur 4. Un troisième état 62 peut correspondre à l'état du capteur bloqué avec un déblocage inhibé. Ce troisième état 62 correspond à une utilisation sécurisée du capteur 4, une action d'un opérateur sur l'ordinateur 1 est nécessaire afin de supprimer l'inhibition. Le capteur 4 peut être mis dans ce troisième état 62 lorsqu'il est peu utilisé par exemple.

Lorsque le capteur 4 est dans l'état 60 débloqué et qu'il est posé sur la station d'accueil 3 :
■ soit il passe dans l'état 61 capteur bloqué et déblocage non inhibé,
■ soit il passe dans l'état 62 capteur bloqué et déblocage inhibé si la station 3 a reçu préalablement à la pose du capteur 4 un message d'inhibition 45 venant de l'ordinateur 1.

Lorsque le capteur 4 est dans l'état 61 capteur bloqué et déblocage non inhibé :
■ soit une simple pression est exercée sur le bouton 5 et le capteur 4 est débloqué, il passe donc à l'état 60 capteur débloqué,
■ soit la station 3 reçoit le message d'inhibition 45 venant de l'ordinateur 1 et le capteur 4 passe alors dans l'état 62 capteur bloqué et déblocage inhibé.

Lorsque le capteur 4 est dans l'état 62 capteur bloqué et déblocage inhibé :
■ soit l'utilisateur envoie par l'intermédiaire de l'ordinateur 1 un message de désinhibition 49, ce qui fait passer le capteur à l'état 61 capteur bloqué et déblocage non inhibé,
■ soit il est impossible d'envoyer un message de désinhibition 49 et dans ce cas il est nécessaire d'avoir recours à une clef afin de libérer mécaniquement le capteur 4 de la station 3. Le capteur 4 se retrouve alors dans l'état 60 capteur débloqué.

Dans le cadre d'une utilisation standard, le principal avantage de la station selon l'invention est qu'il n'y a pas de partage de clefs entre différents utilisateurs mais seulement une utilisation partagée du système de gestion du matériel d'imagerie médicale existant de manière indépendante de la protection antivol.

Un autre avantage de la station selon l'invention est qu'en utilisation standard il n'est pas nécessaire d'entrer un code ni même de posséder une clef pour chaque utilisation du capteur 4. Ce qui est avantageux par exemple lors d'une utilisation intensive du capteur 4 : les manoeuvres à effectuer pour débloquer le capteur 4 sont alors rapides et simples.

Le blocage du capteur se fait avantageusement par simple pose du capteur 4 sur la station d'accueil 3, sans autre manipulation. Ceci permet de garantir le blocage du capteur 3 dès l'instant où il est posé sur la station d'accueil 3.

Un autre avantage de l'invention est qu'elle est simple à mettre en oeuvre. En effet, l'invention nécessite peu d'ajout sur le matériel existant.

## Revendications

1. Station d'accueil pour un équipement portable capteur de rayons X (4), ledit capteur de rayon X (4) prévu pour être en contact avec la station d'accueil (3) comportant des moyens de fixation (20) de l'équipement portable capteur de rayons X (4) sur la station d'accueil (3), ladite station d'accueil comportant un système électromécanique (48) commandant le déblocage des moyens de fixation (20) à partir d'un signal électrique (44), **caractérisé en ce que** le signal électrique (44) étant mis à l'état inhibé par une commande (45) fournie par une unité logicielle (1).

2. Station d'accueil selon la revendication 1 **caractérisé en ce que** la mise à l'état inhibé du signai électrique (44) est réalisée à partir d'un message d'inhibition (45) envoyé par l'unité logicielle (1) à la station d'accueil (3), ledit message d'inhibition (45) produisant un signal, amplifié (51) par une interface adaptée (46), le signal amplifié ouvrant un circuit d'alimentation (41) du système électromécanique (48).

3. Station d'accueil selon les revendications 1 et 2 **caractérisé en ce qu'**une désinhibition du signal électrique est réalisée à partir d'un message de désinhibition (49) envoyé par l'unité logicielle (1) à la station d'accueil (3), ledit message de désinhibition (49) produisant un signal amplifié par une interface adaptée, le signal amplifié fermant le circuit d'alimentation (41) du système électromécanique (48).

4. Station d'accueil selon la revendication 3 **caractérisé en ce que** la saisie d'un mot de passe par l'intermédiaire de l'unité logicielle (1) entraîne l'envoi du message de désinhibition (49) par l'unité logicielle (1) et sécurise l'utilisation de l'équipement portable capteur de rayons X (4).

5. Station d'accueil selon les revendications 2 à 3 **caractérisé en ce que** l'envoi du message d'inhibition (45) est effectué automatiquement par l'unité logicielle (1) en respectant un critère temporel paramétrable.

6. Station d'accueil selon l'une quelconque des revendications précédentes **caractérisé en ce que** le système électromécanique (48) comporte au moins un électroaimant (27, 28) agissant sur un aimant (26) relié aux moyens de fixation (20), permettant l'ouverture des moyens de fixation (20).

7. Station d'accueil selon l'une quelconque des revendications précédentes **caractérisé en ce que** le signal électrique (44) est produit par un bouton (5) disposé sur la station d'accueil (3).

8. Station d'accueil selon l'une quelconque des revendications précédentes **caractérisé en ce que** le système électromécanique (48) comporte un moyen de déblocage mécanique (50).

9. Station d'accueil selon l'une quelconque des revendications précédentes **caractérisé en ce que** la commande (45) du signal électrique (44) est fournie par un ordinateur (1), comportant un logiciel de gestion de système d'imagerie médicale, relié à la station d'accueil (3) par une liaison (2).

## Claims

1. A docking station for portable x-ray sensor equipment (4), said x-ray sensor (4) being designed to be in contact with the docking station (3), comprising means for affixing (20) the portable x-ray sensor equipment (4) to the docking station (3), said docking station comprising an electromechanical system (48) that controls the unlocking of the affixing means (20) using an electrical signal (44), **characterised in that** the electrical signal (44) is disabled by a command (45) that is provided by a software unit (1).

2. The docking station according to claim 1, **characterised in that** the electrical signal (44) is disabled from a disable message (45) that is sent by the software unit (1) to the docking station (3), said disable message (45) producing a signal that is amplified (51) by a suitable interface (46), the amplified signal opening a supply circuit (41) of the electromechanical system (48).

3. The docking station according to claims 1 and 2, **characterised in that** the electrical signal is enabled from an enable message (49) that is sent by the software unit (1) to the docking station (3), said enable message (49) producing a signal that is amplified by a suitable interface, the amplified signal closing the supply circuit (41) of the electromechanical system (48).

4. The docking station according to claim 3, **characterised in that** entering a password via the software unit (1) causes the enable message (49) to be sent by the software unit (1) and secures the use of the portable x-ray sensor equipment (4).

5. The docking station according to claims 2 to 3, **characterised in that** the enable message (45) is sent automatically by the software unit (1) in accordance with a configurable time criterion.

6. The docking station according to any one of the preceding claims, **characterised in that** the electromechanical system (48) comprises at least one electromagnet (27, 28) that acts on a magnet (26) that is linked to the affixing means (20), allowing the affixing means (20) to be opened.

7. The docking station according to any one of the preceding claims, **characterised in that** the electrical signal (44) is generated by a button (5) that is arranged on the docking station (3).

8. The docking station according to any one of the preceding claims, **characterised in that** the electromechanical system (48) comprises mechanical unlocking means (50).

9. The docking station according to any one of the preceding claims, **characterised in that** the command (45) of the electrical signal (44) is provided by a computer (1) that comprises medical imaging management software and that is linked to the docking station (3) via a link (2).

## Patentansprüche

1. Dockingstation für ein tragbares Röntgensensorgerät (4), wobei der Röntgensensor (4) für den Kontakt mit der Dockingstation (3) ausgelegt ist, umfassend Mittel (20) zum Andocken des tragbaren Röntgensensorgerätes (4) an der Dockingstation (3), wobei die Dockingstation ein elektromechanisches System (48) umfasst, das das Entsperren des Andockmittels (20) mit einem elektrischen Signal (44) steuert, **dadurch gekennzeichnet, dass** das elektrische Signal (44) durch einen Befehl (45) gesperrt wird, der von einer Softwareeinheit (1) gegeben wird.

2. Dockingstation nach Anspruch 1, **dadurch gekennzeichnet, dass** das elektrische Signal (44) durch eine Sperrmeldung (45) gesperrt wird, die von der Softwareeinheit (1) zur Dockingstation (3) gesendet wird, wobei die Sperrmeldung (45) ein Signal erzeugt, das von einer geeigneten Schnittstelle (46) verstärkt wird (51), wobei das verstärkte Signal eine Versorgungsschaltung (41) des elektromechanischen Systems (48) öffnet.

3. Dockingstation nach den Ansprüchen 1 und 2, **dadurch gekennzeichnet, dass** das elektrische Signal von einer Entsperrmeldung (49) entsperrt wird, die von der Softwareeinheit (1) zur Dockingstation (3) gesendet wird, wobei die Entsperrmeldung (49) ein Signal erzeugt, das von einer geeigneten Schnittstelle verstärkt wird, wobei das verstärkte Signal die Versorgungsschaltung (41) des elektromechanischen Systems (48) schließt.

4. Dockingstation nach Anspruch 3, **dadurch gekennzeichnet, dass** die Eingabe eines Passwortes über die Softwareeinheit (1) bewirkt, dass die Entsperrmeldung (49) von der Softwareeinheit (1) gesendet wird und den Gebrauch des tragbaren Röntgensensorgeräts (4) sichert.

5. Dockingstation nach den Ansprüchen 2 bis 3, **dadurch gekennzeichnet, dass** die Entsperrmeldung (45) von der Softwareeinheit (1) automatisch gemäß einem konfigurierbaren Zeitkriterium gesendet wird.

6. Dockingstation nach einem der vorherigen Ansprüche, **dadurch gekennzeichnet, dass** das elektromechanische System (48) wenigstens einen Elektromagnet (27, 28) umfasst, der auf einen Magnet (26) wirkt, der mit dem Andockmittel (20) verbunden ist, so dass das Andockmittel (20) geöffnet werden kann.

7. Dockingstation nach einem der vorherigen Ansprüche, **dadurch gekennzeichnet, dass** das elektrische Signal (44) von einer Taste (5) erzeugt wird, die an der Dockingstation (3) angeordnet ist.

8. Dockingstation nach einem der vorherigen Ansprüche, **dadurch gekennzeichnet, dass** das elektromechanische System (48) mechanische Entsperrmittel (50) umfasst.

9. Dockingstation nach einem der vorherigen Ansprüche, **dadurch gekennzeichnet, dass** der Befehl (45) des elektrischen Signals (44) von einem Computer (1) gegeben wird, der medizinische Bilderzeugungs-Managementsoftware umfasst und der durch eine Verbindung (2) mit der Dockingstation (3) verbunden ist.
